# EUROPÄISCHE PATENTSCHRIFT

(11) **EP 4 017 559 B1**
(45) Veröffentlichungstag und Bekanntmachung des Hinweises auf die Patenterteilung: **10.09.2025**
(21) Anmeldenummer: 20758169.5
(22) Anmeldetag: 18.08.2020
(51) Int. Cl.: A61M 5/315, G01F 11/02, A61M 5/20, G01F 13/00

(54) **ELEKTRONIKMODUL MIT SENSOREINHEIT**
ELECTRONICS MODULE WITH SENSOR UNIT
MODULE ÉLECTRONIQUE DOTÉ D'UNE UNITÉ DE DÉTECTION

(30) Priorität: 20.08.2019 CH 10422019
(43) Veröffentlichungstag der Anmeldung: 29.06.2022
(73) Patentinhaber: Ypsomed AG, 3401 Burgdorf (CH)
(72) Erfinder: STECK, Jürg, 3422 Kirchberg (CH); HIRSCHEL, Jürg, 3007 Bern (CH); KÜHNI, Florian, 3007 Bern (CH)
(74) Vertreter: Kleiner, Stefan
(86) Internationale Anmeldenummer: PCT/EP2020/073053
(87) Internationale Veröffentlichungsnummer: WO 2021/032709

(56) Entgegenhaltungen:
- WO-A1-2019/123257
- US-A1- 2017 312 457
- US-A1- 2019 247 584

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf ein Elektronikmodul mit einer Sensoreinheit zum lösbaren Verbinden mit einem medizinischen Injektionsgerät.

### HINTERGRUND DER ERFINDUNG

Unterschiedliche Infusions- und Injektionsgeräte können Patienten bei einer kontrollierten subkutanen oder intramuskulären Verabreichung einer Dosis eines Medikamentes aus einem Reservoir unterstützen. In Einweg-Injektionsgeräten ist das Reservoir, beispielsweise eine Fertigspritze, nicht dazu vorgesehen, durch den Patienten ersetzt oder wiederaufgefüllt zu werden. In Mehrweg-Injektionsgeräten dagegen ist kann das Reservoir, beispielsweise eine Karpule, durch den Patienten wieder befüllt oder ersetzt werden. Ein automatisches Injektionsgerät verfügt über einen Motor oder eine gespannte Feder als Energiequelle zum Antrieb einer Kolbenstange und zur Bewegung eines Kolbens im Reservoir, während bei einer Ausschüttung mit einem manuellen Injektionsgerät die Kolbenstange unmittelbar durch eine Kraft des Patienten bewegt wird.

Diabetes wird oft durch Abgabe von Insulin mittels eines Injektionsgeräts oder Injektionsvorrichtung in Form eines Insulin-Pens mit einstellbarer Dosis behandelt. Der Pen verfügt über ein längliches Gehäuse mit einem distalen Ende zur Aufnahme einer Kanüle oder Hohlnadel und mit einem der Injektionsstelle abgewandten proximalen Ende. Ein Insulin-Pen kann als Ein- oder Mehrweg-Pen, automatisch oder manuell betrieben werden. Die abzugebende Insulindosis wird normalerweise eingestellt durch Drehen eines Dosierknopfs und gleichzeitiger Kontrolle einer Dosisanzeige des Insulin-Pens.

Aus dem Stand der Technik sind Elektronikmodule bekannt, welche lösbar mit einem Injektionsgerät verbunden werden können. Solche Elektronikmodule sind wiederverwendbar und können mit einem Einweginjektor wie beispielsweise einen Autoinjektor oder mit einem Mehrweginjektor verbunden werden. Die Elektronikmodule werden auch als Zusatzmodul, "Smart device", "Smart module" oder "Add-on" bezeichnet. Ein Elektronikmodul dient dazu Vorgänge, Ereignisse und Funktionen des Injektionsgeräts zu erkennen, zu überwachen und erfasste Ereignisse dem Anwender zu melden oder an einen externen Empfänger zu senden. So kann zum Beispiel der Anwender beim Injektionsvorgang in Echtzeit direkt über eine Anzeige auf dem Elektronikmodul unterstützt werden, indem Instruktionen für die Injektion, Vorschläge für die Injektionszeit und/oder Handhabungsfehler angezeigt werden. Ferner können mittels des Elektronikmoduls visuelle und akustische Rückmeldung für spezielle Ereignisse wie zum Beispiel das erfolgreiche Abschliessen einer Injektion ausgegeben werden. Das Elektronikmodul kann zudem Informationen über getätigte Injektionen aufzeichnen und weiterleiten oder anhand der aufgezeichneten Daten Auswertungen vornehmen.

Die Patentanmeldung US 2017/182258 A1 offenbart beispielswese ein Elektronikmodul, welches lösbar mit einem Einweginjektor verbindbar ist. Das Elektronikmodul kann mittels Piezosensoren die beim Einstellen einer Dosis bei jedem Dosisinkrement erzeugten Vibrationen erkennen und daraus die eingestellte Dosis berechnen. Der Sensor kann dabei erkennen, wenn eine Vibration nicht eindeutig ist und die Dosis somit nicht eindeutig bestimmbar ist. Das Elektronikmodul gibt in diesem Fall eine Warnung aus und fordert Benutzer auf, die Dosis manuell zu überprüfen oder am Elektronikmodul eine Kalibrierung vorzunehmen.

US 2017/312457 A1 offenbart einen Autoinjektor, welcher eine Kolbenstange mit einem farbigen Indikator umfasst, um eine noch verbleibende Menge Medikament im Autoinjektor anzuzeigen. Ist der Autoinjektor noch voll, ist durch ein Sichtfenster eine schwarze Farbe erkennbar. Im ausgeschütteten Zustand ist nur eine graue Farbe ersichtlich. Mittels eines Aufnahmegeräts, welches ein Bild des Autoinjektor aufnehmen kann, kann anhand des Indikators ein Zustand des Autoinjektors festgestellt werden.

US 2019/0247584 A1 offenbart einen Injektionspen mit einer Dosierhülse mit weissen Dosiswerten auf schwarzem Untergrund. Zudem weisen Dosiswerte, welche in einem zulässigen Dosisbereich liegen eine andere Farbe auf als Dosiswerte, welche in einem unzulässigen Bereich liegen. Ein optischer Sensor in einem aufsetzbaren Zusatzmodul (Add-On) kann die zwei Farben der Dosiswerte unterscheiden und den eingestellten Dosiswert entsprechend auswerten.

WO 2019/123257 A1 offenbart einen Injektionspen mit einer Kappe, welche im Innern einen optischen Sensor umfasst, welcher reflektierendes farbiges Licht erkennen kann. Der Sensor kann dadurch optisch eine Position oder eine Bewegung der Kolbenstange des Injektionspens detektieren.

Die Patentanmeldung WO 2013/004844 A1 beschreibt ein aufsetzbares Elektronikmodul, welches eine bewegliche Sensorscheibe umfasst, die zusammen mit einem drehbaren Dosiereinstellelement des Injektionspens rotiert. Auf der beweglichen Sensorscheibe befindet sich ein Gray-Code, wodurch mittels eines optischen Sensors eine Absolutposition des Dosiereinstellelements und damit einen Absolutwert einer eingestellten Dosis bestimmt werden kann.

Solche mitdrehenden Sensorscheiben ermöglichen mittels eines bestimmten Codes zwar eine absolute Bestimmung der Dosis. Sie benötigen jedoch viel Platz, wodurch das Bauvolumen des Elektronikmoduls entsprechend gross ausfällt.

### DARSTELLUNG DER ERFINDUNG

Es ist eine Aufgabe der Erfindung auf einfache und platzsparende Weise ein Bestimmen eines absoluten Dosiswertes bei einem Injektionsgerät zu ermöglichen. Diese Aufgabe wird gelöst durch ein Elektronikmodul und durch ein Injektionssystem mit den Merkmalen der unabhängigen Ansprüche. Bevorzugte Ausführungsformen der Erfindung sind Gegenstand der abhängigen Ansprüche.

Gemäss der Erfindung umfasst ein lösbar mit der Injektionsvorrichtung verbindbares Elektronikmodul eine Sensoreinheit mit einem Farbsensor, welcher dazu ausgebildet ist, mindestens eine erste, eine zweite und eine dritte Farbe einer optischen Markierung eindeutig zu unterscheiden und eindeutig zu erkennen. Weiter ist die Sensoreinheit dazu ausgebildet, mittels vorbekannten Daten und basierend auf der eindeutig erkannten Farbe einen absoluten Wert der eingestellten Dosis zu bestimmen.

Zudem umfasst die Erfindung ein Injektionssystem umfassend eine Injektionsvorrichtung und das Elektronikmodul mit der Sensoreinheit, wobei die Injektionsvorrichtung ein proportional zu einer einstellbaren Dosis bewegliches Element mit der optischen Markierung umfasst.

Die Sensoreinheit des Elektronikmoduls umfasst einen Farbsensor, welcher mindestens drei unterschiedliche Farben voneinander unterscheiden und jede Farbe eindeutig erkennen kann. Mittels vorbekannten Daten wie beispielsweise einer Datentabelle (Look-up-Tabelle) kann die Sensoreinheit die erfasste Farbe einem bestimmten Dosierwert zuordnen. Das bedeutet, in einem Speicher des Elektronikmoduls ist eine Zuordnung hinterlegt, die jedem einstellbaren Dosiswert eindeutig eine Farbe zuordnet. Dadurch kann die Sensoreinheit jederzeit anhand der erfassten Farbe der Markierung ein vom Benutzer an der Injektionsvorrichtung eingestellter absoluter Wert der Dosis bestimmen.

Durch diese Möglichkeit der Absolutwertbestimmung entfällt ein Kalibrieren oder ein manuelles Zurücksetzen eines Zählers der Sensoreinheit. Ein solcher Schritt ist beispielsweise nach einem Stromausfall oder nach einem Wechsel des Elektronikmoduls von einer Injektionsvorrichtung zur nächsten nötig, wenn die Sensoreinheit, wie häufig üblich, einzelne Dosierschritte mittels Detektion eines Klickgeräusches oder einer Vibration zählt und anschliessend die eingestellte Dosis berechnet (relative Dosisbestimmung). Durch die Bestimmung mittels Farben kann die Sensoreinheit auch bei einem Unterbruch, nach einer Fehlmanipulation oder nach einem Wechsel der Injektionsvorrichtung die momentane absolute Position des Dosierelements und damit die eingestellte Dosis zuverlässig bestimmen.

Der Farbsensor ist dazu ausgebildet, eine spezifische Farbe oder einen bestimmten Farbton zu erkennen und ein entsprechendes Sensorsignal zu liefern, welches von der Sensoreinheit ausgewertet werden kann. Der Farbsensor kann mindestens drei, vorzugsweise mindestens vier unterschiedliche Farben voneinander unterscheiden und erkennen. In einer besonders bevorzugten Ausführung kann der Farbsensor mindestens 20, insbesondere mindestens 80 unterschiedliche Farben eindeutig unterscheiden und erkennen. Somit unterscheidet sich der Farbsensor von Sensoren, welche nur zwischen schwarz und weiss unterscheiden können und verwendet werden zum Einlesen einer Schwarz-Weiss-Kodierung wie beispielsweise eines Gray-Codes. Das erfindungsgemässe Elektronikmodul mit dem Farbsensor benötigt für die Bestimmung eines Absolutwertes also nicht einen ganzen Code, sondern kann den Absolutwert der eingestellten Dosis anhand der erkannten Farbe eindeutig bestimmen.

Unter dem Begriff "Farbe" oder "Farbton" werden in der vorliegenden Beschreibung einerseits bestimmte Intervalle von Wellenlänge, welche durch das menschliche Auge unterscheidbar sind, und andererseits gleiche oder ähnliche Intervalle von Wellenlänge, welche mit einer unterschiedlichen Intensität oder Farbtemperatur wahrgenommen werden, verstanden. Demnach sind zwei unterschiedliche Farben beispielsweise "blau" und "grün". Aber auch "hellblau" und "dunkelblau" werden in der vorliegenden Anmeldung als unterschiedliche Farben verstanden. Ferner umfasst der Begriff "Farbe" in dieser Beschreibung auch weiss, grau oder schwarz, obwohl diese keine Spektralfarben sind.

Unter absoluten Wert der Dosis oder der Absolutwertbestimmung wird ein spezifischer und eindeutiger Wert oder eine Grösse einer Dosis verstanden. Demgegenüber ist eine Bestimmung der Dosierung mittels Messung von einzelnen Inkrementen, Einstell- oder Dosierschritten eine relative Bestimmung der Dosierung, beispielsweise indem die einzelnen Dosierinkremente zusammengezählt werden und mittels der Summe der eingestellte Dosierwert berechnet wird.

Das Elektronikmodul kann unterschiedliche Injektionsereignisse, welche mit dem Gebrauch der Injektionsvorrichtung auftreten, erfassen. Die Injektionsereignisse können auch als Vorgänge in der Injektionsvorrichtung während dem Gebrauch bezeichnet werden. Solche Injektionsereignisse sind beispielsweise das Einstellen und Korrigieren einer Dosis, das Ausschütten einer eingestellten Dosis, das Einstellen oder Primen der Injektionsvorrichtung, das Erfassen von Zeit und Datum von solchen Ereignissen, aber auch das Austauschen einer Karpule in einem Mehrweg-Injektor.

Die Markierung ist vorzugsweise auf einem beweglichen Element der Injektionsvorrichtung angebracht, welches sich beim Einstellen und Korrigieren und je nach Ausführung optional auch beim Ausschütten relativ zum Elektronikmodul bzw. zum Farbsensor oder zur Sensoreinheit und damit vorzugsweise auch relativ zu einem Gehäuse der Injektionsvorrichtung bewegt. Dabei kann die Bewegung eine Verschiebung, eine Rotation oder auch eine Schraubbewegung des beweglichen Elements relativ zum Gehäuse der Injektionsvorrichtung sein. Die Markierung kann beispielsweise an einem Dosierelement, wie einer Dosierhülse, an einem Einstellknopf, an einer Kolbenstange oder an einem sonstigen Element der Dosier- oder Antriebseinrichtung der Injektionsvorrichtung angeordnet sein. Hierzu ist die Markierung vorzugsweise umlaufend auf einem zylindrischen Element, also kreisrund oder linear, spiralförmig oder schraubenlinienförmig auf einem Element angeordnet.

Die Markierung umfasst mindestens drei unterschiedliche Farben, vorzugsweise mindestens vier unterschiedliche Farben, wobei in einer bevorzugten Ausführung jede Farbe eindeutig eine bestimmte Dosierung repräsentiert oder kennzeichnet. In einer bevorzugten Ausführung wird die Markierung aus einzelnen diskreten Farben gebildet. Alternativ besteht aber auch die Möglichkeit, dass die Markierung durch einen oder mehrere Farbverläufe gebildet wird, bei denen eine Farbe kontinuierlich mittles Farbverlauf in eine andere Farbe übergeht. Weiter kann beispielsweise jeder Zahlenwert auf einem Dosierelement in einer eigenen Farbe dargestellt werden. Da vorzugsweise jede Farbe eindeutig einem bestimmten Dosierwert zugeordnet ist, kann die Sensoreinheit nach dem Erfassen der Farbe den entsprechenden eindeutigen Dosierwert aus den vorbekannten und abgespeicherten Daten oder der Datenbank auslesen und dem Benutzer beispielsweise über eine Anzeigeeinheit am Elektronikmodul anzeigen oder an einen externen Empfänger senden.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Injektionsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Injektionsvorrichtung hin gerichtete Seite oder Richtung.

Die Injektionsvorrichtung kann ein Einweg- oder Mehrweginjektionvorrichtung sein. Sie dient zum Ausschütten einer eingestellten Dosis eines Produkts mittels manuellem Vorschub einer Kolbenstange in eine in der Injektionsvorrichtung gehaltenen Karpule. Die Injektionsvorrichtung kann dabei stiftförmig wie ein Injektionspen oder auch rechteckförmig wie ein Patch-Injektor ausgebildet sein. Der Injektionspen wird üblicherweise vom Benutzer während der Injektion gehalten und nach Abschluss der Abgabe wieder von der Injektionsstelle entfernt. Der Patch-Injektor kann einen selbstklebenden Bereich aufweisen, mit dem er auf oder neben der Injektionsstelle auf der Haut aufgeklebt werden kann. Dadurch kann der Patch-Injektor auch über einen längeren Zeitraum von mehreren Minuten auf der Haut verbleiben.

Unter den Begriffen "Injektionssystem" oder "Injektionsvorrichtung" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einer Injektionsvorrichtung im Unterschied zu einem Infusionssystem die Injektionsnadel nicht über einen längeren Zeitraum von mehreren Stunden im Gewebe.

In einer bevorzugten Ausführung umfasst die Sensoreinheit im Elektronikmodul weiter eine Lichtquelle, mit welcher eine Oberfläche der zu erfassenden Markierung am Dosiseinstellelement der Injektionsvorrichtung beleuchtet werden kann. Die Lichtquelle ist dabei vorzugsweise eine LED. Indem die Lichtquelle die unterschiedlichen Farben der Markierung mit Licht beleuchtet, kann die Sensoreinheit die eingestellt Dosis auch in dämmrigem oder schwachem Umgebungslicht zuverlässig erfassen. Das Licht ist dabei vorzugsweise weisses Licht. Alternativ kann die Lichtstrahlung aber auch eine beliebige Wellenlänge aufweisen.

Weiter umfasst in einer bevorzugten Ausführung die Sensoreinheit zudem eine Linse, um das von der Lichtquelle ausgesendete Licht auf der Oberfläche gleichmässig zu verteilen oder um das Licht auf einen Bereich der Oberfläche zu fokussieren. Wenn das Licht der Lichtquelle durch die Linse verteilt oder zerstreut wird, weist die Linse vorzugsweise optische Streuelemente auf, so dass die Oberfläche durch ein diffuses und gleichmässiges Licht beleuchtet wird. Wird das Licht mittels der Linse fokussiert, kann ein spezifischer Lichtkegel erreicht werden, der einen bestimmten Bereich der Oberfläche ausleuchten kann.

Somit kann mittels der Linse die Markierung durch die Lichtquelle in optimaler Weise beleuchtet werden. Dadurch kann die Erfassung der Farben durch den Farbsensor weiter verbessert werden. So können beispielsweise zwei unterschiedliche Farben, welche sich aber sehr ähnlich sind, zuverlässig erfasst werden. Die Linse ist vorzugsweise zwischen der Lichtquelle und der Markierung angeordnet. Alternativ oder zusätzlich kann auch eine Linse zwischen der Markierung und dem Farbsensor vorgesehen werden, beispielsweise um die Markierung für den Farbsensor zu vergrössern.

Dabei sind vorzugsweise der Farbsensor, die Lichtquelle und die eine Linse oder mehrere Linsen auf einer gemeinsamen Grundplatte angeordnet. Dadurch sind Farbsensor, Lichtquelle und Linse einfach relativ zueinander positionierbar. Zudem können Farbsensor, Lichtquelle und Linse auf der Grundplatte vormontiert werden, was die Herstellung der Sensoreinheit und die Montage der Sensoreinheit im Elektronikmodul vereinfacht.

Vorzugsweise handelt es sich beim Farbsensor um einen Rot-Grün-Blau-Sensor (RGB-Sensor). Der RGB-Sensor kann über die Intensität des Farbanteils die jeweilige Farbe auf dem Dosierelement erfassen. Mittels des RGB-Sensors können die unterschiedlichen Farben der Markierung zuverlässig erfasst und damit die eingestellte Dosis zuverlässig bestimmt werden. Dabei können handelsübliche RGB-Sensoren verwendet werden, welche problemlos verfügbar und kostengünstig erhältlich sind.

In einer bevorzugten Ausführung umfasst das Elektronikmodul weiter eine Kommunikationseinheit, welche mit der Sensoreinheit verbunden ist und dazu ausgebildet ist, den erfassten absoluten Wert drahtlos an einen externen Empfänger zu übermitteln. Die erfassten Daten von einem Injektionereignis wie beispielsweise die eingestellte Dosis, der Zeitpunkt der Verabreichung und das verwendete Medikament sowie Angaben zur Injektionsvorrichtung können somit an einen externen Empfänger gesandt werden, welcher die gesammelten Daten weiter auswerten kann. Der Empfänger kann beispielsweise ein mobiles Benutzergerät wie ein Smart Phone, ein Computer oder auch eine Daten-Cloud, ein Server oder eine medizinische Einrichtung sein. Die Übermittlung kann je nach Distanz via Nahfeldkommunikation wie NFC, Bluetooth oder Infrarot oder über ein lokales oder öffentliches Drahtlosnetzwerk wie ein WLAN, WAN oder Internet erfolgen.

Bevorzugt kann die Kommunikationseinheit zusätzlich Daten von einem externen Sender empfangen. So können zum Beispiel Injektionsdaten an einen Server gesendet werden. Dieser vergleicht die verabreichte Dosis und der Zeitpunkt mit einem benutzerspezifischen Therapieplan und senden eine Rückmeldung an das Elektronikmodul, welches die Rückmeldung dem Benutzer anzeigt. Das Elektronikmodul weist zu diesem Zweck vorzugsweise ein Display auf. So kann beispielsweise bestätigt werden, dass der Benutzer die richtige Dosis verabreicht oder der Benutzer kann auf eine zu hoch oder zu tief eingestellte Dosis hingewiesen werden.

Dabei repräsentiert bei der Injektionsvorrichtung vorzugsweise jede Farbe der Markierung einen einstellbaren absoluten Dosierwert. Die Markierung umfasst somit vorzugsweise für jeden einstellbaren Dosierwert eine eigene Farbe. Mit anderen Worten ausgedrückt, umfasst die Markierung vorzugsweise jede Farbe nur einmal, so dass jede Farbe eindeutig einem Dosierwert zugeordnet werden kann. Wenn die Einstellung der Dosis verändert wird, ändert auch die von aussen ersichtliche Farbe. Wie oben erwähnt, kann beispielsweise die Zahl der jeweiligen Dosierung in der Farbe dargestellt werden oder eine farbliche Markierung kann anstelle oder neben der Zahl dargestellt sein.

Weiter kann die Zuordnung der Dosierwerte zu einer Farbe je nach Ausführung der Injektionsvorrichtung oder je nach Benutzer individuell vorgenommen werden. Der Farbsensor kann also mittels Kalibrierung auf einen bestimmten Farbverlauf oder auf eine bestimmte Anzahl Farben eingestellt werden.

In einer bevorzugten Ausführung ist jeder Farbe der Markierung ein eindeutiger Code, insbesondere eine eindeutige Nummer, zugeordnet. Der Farbsensor ist dazu ausgebildet anhand einer Reihenfolge von Codes von erfassten Farben einen durch die Sensoreinheit bestimmten absoluten Wert einer eingestellten Dosis zu verifizieren. Die Zuordnung der Codes der Farben ist dabei vorzugsweise in der Sensoreinheit abgespeichert. Es kann somit eine Plausibilitätsprüfung erfolgen, ob der durch die Sensoreinheit bestimmte absolute Dosierwert korrekt ist. Das kann beispielsweise dadurch erfolgen, dass ähnlichen Farben oder im Farbspektrum nebeneinanderliegende Farben Zahlenwerte zugeordnet sind, welche ebenfalls nebeneinander liegen. So kann beispielsweise erkannt werden, wenn Zahlenwerte von erfassten Farben nicht einer erwarteten Reihenfolge oder einem erwarteten Muster (z.B. aufsteigende Zahlen) entsprechen. In diesem Fall kann ein Fehler in der Erfassung der Farbe erkannt und dem Benutzer gemeldet werden.

Bevorzugt ist die Markierung auf einem Dosierelement angeordnet. Dieses ist vorzugsweise bewegbar relativ zu einem Gehäuse der Injektionsvorrichtung. Die Bewegung des Dosierelements relativ zum Gehäuse kann eine Verschiebe- Rotations- oder Schraubbewegung sein. In einer bevorzugten Ausführung ist das Dosierelement eine Dosierhülse, welche in einer Gewindeverbindung mit dem Gehäuse der Injektionsvorrichtung steht. Die Markierung ist in diesem Fall vorzugsweise auf einer Aussenseite umlaufend auf der Dosierhülse angeordnet.

Alternativ dazu kann das Dosierelement beispielsweise auch eine Dosierstange sein, welche zum Dosieren aus dem Gehäuse gezogen wird, oder ein Dosierknopf, welcher relativ zu Gehäuse rotiert wird, oder eine Dosierscheibe, welche um die Gehäuseachse oder geneigt zu dieser rotiert werden kann, zum Einstellen einer Dosis.

Bevorzugt umfasst das Gehäuse der Injektionsvorrichtung eine Öffnung, wobei durch die Öffnung die Markierung von aussen ersichtlich ist und wobei im verbundenen Zustand der Farbsensor des Elektronikmoduls über der Öffnung angeordnet ist. Dadurch kann der Farbsensor die Farben der Markierung auf dem Dosierelement, welches sich im Gehäuse befindet, in optimaler Weise auslesen.

Alternativ dazu besteht auch die Möglichkeit, dass im verbundenen Zustand (Elektronikmodul ist auf der Injektionsvorrichtung aufgesetzt) der Farbsensor an einem proximalen Ende des Elektronikmoduls angeordnet ist und ein aus dem Gehäuse der Injektionsvorrichtung herausragendes Dosierelement erfassen kann.

In einer weiteren Ausführung umfasst die Injektionsvorrichtung eine Kolbenstange mit einer Positionsmarkierung mit mindestens drei unterschiedlichen Farben, vorzugsweise mindestens vier unterschiedliche Farben, und das Elektronikmodul umfasst zusätzlich zur oben beschriebenen Sensoreinheit eine zweite Sensoreinheit zum Erfassen einer absoluten Position der Kolbenstange. Die zweite Sensoreinheit umfasst einen zweiten Farbsensor, vorzugsweise ein RGB-Farbsensor, welcher dazu ausgebildet ist, mindestens eine erste, eine zweite und eine dritte Farbe und vorzugsweise eine vierte Farbe der Positionsmarkierung zu erkennen und wobei die zweite Sensoreinheit dazu ausgebildet ist, mittels vorbekannten Positionsdaten und basierend auf der erkannten Farbe eine absolute Position der Kolbenstange zu bestimmen.

Wie oben in Zusammenhang mit der ersten Sensoreinheit beschrieben, kann auch mit der zweiten Sensoreinheit anhand der erfassten Farbe auf der Kolbenstange mittels vorbekannten Positionsdaten, beispielsweise einer Zuordnungstabelle, welche eine bestimmte Farbe einer Positionsangabe zuordnet, eine eindeutige Position der Kolbenstange relativ zu einem Gehäuse der Injektionsvorrichtung bestimmt werden. Hierzu sind die unterschiedlichen Farben auf der Kolbenstange hintereinander bzw. nebeneinander so angeordnet, dass jede Farbe für eine bestimmte und eindeutige Position der Kolbenstange relativ zum Gehäuse steht. Die Zuordnungstabelle oder Look-Up-Tabelle ist dabei bevorzugt im Elektronikmodul abgespeichert.

In einer bevorzugten Ausführung kann anhand der erfassten Position zudem das verbleibende Volumen in der Karpule bestimmt werden, da abhängig von der Kolbenstangenposition bestimmbar ist, wie weit ein Stopfen der Karpule in distaler Richtung in die Karpule eingeschoben worden ist und somit wie gross das verbleibende Volumen mit dem Produkt in der Karpule ist. Die verbleibende Menge in der Karpule kann anschliessend durch das Elektronikmodul dem Benutzer angezeigt werden oder an einen externen Empfänger weitergeleitet werden. Neigt sich der Inhalt der Karpule dem Ende zu kann weiter beispielsweise dem Benutzer eine Meldung angezeigt werden oder eine Nachricht an einen externen Empfänger gesandt werden.

In dieser Ausführung wird also einerseits mittels der ersten Sensoreinheit die eingestellte Dosierung erfasst und mit der zweiten Sensoreinheit andererseits die Position der Kolbenstange relativ zu einem Gehäuse der Injektionsvorrichtung erfasst. Dadurch besteht anhand der erfassten Dosierungen und der erfassten Position der Kolbenstange die Möglichkeit, eine Plausibilitätsprüfung der Sensorsignale und/oder Messwerte durchzuführen.

In einer weiteren nicht erfindungsgemässen Ausführung kann die Sensoreinheit anstelle eines oben beschriebenen Farbsensors ein Sensorchip-Set umfassen, welches für eine Computermaus verwendet wird. Solche Chipsets werden auch als "Optical Mouse Chipset" bezeichnet. Dieses Set umfasst einen CCD-Bildsensor, eine Lichtquelle in Form einer LED und eine Optik mit Linsen, um das Licht der Lichtquelle auf die zu erfassenden Oberfläche zu projiziern, zu fokusieren oder um das Licht zu zerstreuen. Der CCD-Bildsensor kann eine Bewegung des Dosierelements erfassen und ermöglicht durch das Erfassen von Dosisinkrementen eine relative Dosisbestimmung.

In einer weiteren nicht erfindungsgemässen Ausführung kann die Sensoreinheit einen Linienscanner mit einem CCD-Bildsensor umfassen, mit dem ein auf dem Dosierelement angeordneter Code, beispielsweise ein Barcode, eingelesen werden kann. Je nach Ausführung des Codes kann dabei eine absolute Dosisbestimmung oder durch Zählen von einzelnen erfassten Dosisinkrementen eine relative Dosisbestimmung erfolgen.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt eine schematische Darstellung eines mit einem Injektionspen verbundenen Elektronikmoduls, wobei dieses entlang seiner Längsachse geschnitten dargestellt ist;
- Fig. 2: zeigt eine schematische und vereinfachte Darstellung der Sensoreinheit;
- Fig. 3: zeigt eine weitere Ausführung des Elektronikmoduls mit einer zweiten Sensoreinheit.

### FIGURENBESCHREIBUNG

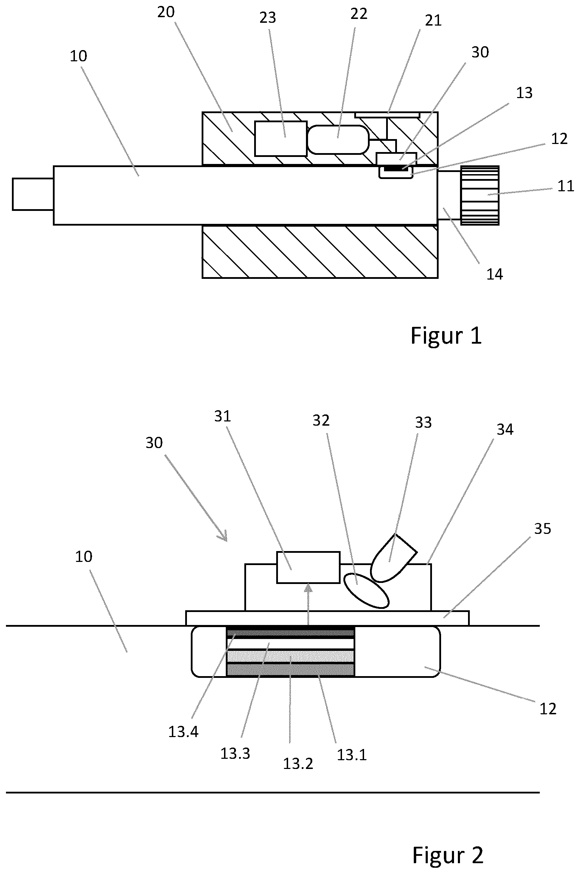

In der Figur 1 ist ein schematisches, nicht massstabsgetreu gezeichnetes erfindungsgemässes Elektronikmodul 20 dargestellt, welches mit einem Einweginjektionspen 10 verbunden ist. In dieser schematischen Darstellung ist das Elektronikmodul 20 entlang seiner Längsrichtung geschnitten dargestellt. Das distale, einstechseitige Ende befindet sich in Figur 1 auf der linken Seite. Das proximale Ende ist entsprechend auf der rechten Seite.

Der Injektionspen 10 umfasst ein Gehäuse, eine in diesem drehbar gelagerte Dosierhülse 14 als Dosiseinstellelement und eine Antriebseinheit zum Ausschütten der eingestellten Dosis (nicht ersichtlich). Die Dosierhülse 14 umfasst am proximalen Ende einen Dosierknopf 11, an dem der Benutzer die Dosierhülse 14 relativ zum Gehäuse drehen kann, um eine Dosis einzustellen oder zu korrigieren. Weiter weist die Dosierhülse 14 auf ihrer Aussenseite über den Umfang angeordnet eine Zahlenskala mit einer mehrfarbigen Markierung 13 auf. Dabei steht jeder Zahlenwert zusammen mit einer Farbe für einen bestimmten Dosierwert.

Das Gehäuse des Injektionspens 10 weist eine radiale Öffnung 12 auf, durch die jeweils ein Zahlenwert der Zahlenskala und eine Farbe der Markierung 13 von aussen ersichtlich sind. Wenn der Benutzer durch Drehen der Dosierhülse 14 die Dosis einstellt, ändert sich die Anzeige. Dabei kann die Farbe, wie weiter unten im Detail erläutert, neben der Zahl für den Dosierwert angeordnet sein, oder die Zahlen selber können sich farblich unterscheiden.

Das Elektronikmodul 20 weist eine in axialer Richtung durchgehende Öffnung auf. Indem der Injektionspen 10 in die Öffnung eingeschoben wird, kann das Elektronikmodul lösbar mit dem Injektionspen 10 verbunden werden. Mittels elastischen Schnapper des Elektronikmoduls 20, welche in entsprechende Aussparungen (nicht dargestellt) im Gehäuse des Injektionspens 10 eingreifen, wird das Elektronikmodul 20 rotativ wie auch axial am Gehäuse gehalten werden. Das Elektronikmodul 20 hat vorzugsweise auf seiner Aussenseite eine Grifffläche, an welcher der Benutzer das Elektronikmodul 20 zusammen mit dem Injektionspen 10 ergreifen kann.

Zudem umfasst das Elektronikmodul 20, wie in Figur 2 ersichtlich, eine Sensoreinheit 30 mit einem Farbsensor 31, eine Auswerteinheit 22, eine Kommunikationseinheit 23 und ein Display 21 zum Anzeigen von erfassten Injektionsereignissen wie beispielsweise eine erfasste, eingestellte Dosis. Wenn, wie in Figur 1 schematisch gezeigt, das Elektronikmodul 20 mit dem Injektionspen 10 verbunden ist, befindet sich der Farbsensor 31 direkt über der radialen Öffnung 12 des Injektionspens 10. Dadurch kann der Farbsensor 31 die jeweils angezeigte Farbe auslesen. Anhand dieser kann die Sensoreinheit 30, wie im Zusammenhang mit Figur 2 weiter unten beschrieben, die eingestellte Dosis bestimmen.

Die Figur 2 zeigt schematisch und vereinfacht eine vergrösserte Ansicht der Sensoreinheit 30 aus Figur 1. Der Übersichtlichkeit halber ist nur die Sensoreinheit 30 dargestellt und es sind keine weiteren Elemente des Elektronikmoduls 20 abgebildet. Ersichtlich in Figur 2 ist der Bereich des Injektionspens 10 mit der radialen Öffnung 12 im Gehäuse, durch die die farbige Markierung 13.1-13.4 von aussen ersichtlich ist. Die Zahlenskala für den Dosierwert ist nicht dargestellt. Wie jedoch in Figur 2 erkennbar, weist die Dosierhülse über den Umfang angeordnet pro Dosisinkrement je eine farbige Markierungen 13.1 - 13.4 auf. Je nach rotativer Position der Dosierhülse 14 und damit je nach eingestellter Dosis befindet sich eine andere Farbe direkt unter dem Farbsensor 31, welche vom Farbsensor 31 erfassbar ist (siehe Pfeil in Figur 2).

Die Sensoreinheit 30 umfasst einen RGB-Farbsensor 31, eine LED 33 als Lichtquelle, eine erste Linse 32 als Lichtdiffusor, eine zweite Linse 36 für den Farbsensor und eine Grundplatte 35, auf der mittels einer Halterung 34 der Farbsensor 31, die LED 33 und die Linsen 32, 36 relativ zueinander positioniert und gehalten sind. Dabei ist die zweite Linse 36 nicht zwingend, sie kann je nach Farbsensor 31 auch entfallen. Die Grundplatte 35 weist dabei eine durchgehende Bohrung auf, durch die der Farbsensor 31 die auf der Dosierhülse 14 befindliche farbige Markierung 13.1 - 13.4 lesen kann. Die Linse 32 weist optische Streuelemente auf und verteilt oder zerstreut das von der LED 33 ausgesendete vorzugsweise weisse Licht auf die Oberfläche der Dosierhülse 14, so dass die Farbe der Markierung durch den Farbsensor 31 zuverlässig erfasst werden kann. Dabei ist die Reihenfolge der Farbtöne so gewählt, dass bei einem Dosierschritt vorzugsweise nur ein Farbanteil (rot, gelb oder grün) verändert wird. Dabei ist jeder Farbe ein eindeutiger Zahlenwert zugeordnet, wobei im Farbspektrum nebeneinanderliegende Farben Zahlenwerte aufweisen, die betragsmässig ebenfalls nebeneinander liegen. Die Zahlenwerte für jede Farbe und eine vorbestimmte Reihenfolge der Zahlenwerte sind in der Sensoreinheit 30 abgespeichert. Beim Dosieren und Korrigieren einer Dosis werden die Zahlenwerte aus den erfassten Farben in einer bestimmten Reihenfolge durchlaufen. Das Sensorsignal des RGB-Farbsensors 31 kann dadurch besonders einfach auf Plausibilität überprüft werden. Das heisst, wenn von der erwarteten Zahlenwertreihenfolge abgewichen wird, ist von einer falsch erfassten Farbe auszugehen und dem Benutzer kann eine entsprechende Warnung angezeigt werden.

Wird die Sensoreinheit 30 eingeschaltet bzw. aktiviert, erfasst der Farbsensor 31 die Farbe auf der Dosierhülse 14. In der Auswerteinheit 22 wird das Sensorsignal ausgewertet und die jeweilige Farbe bestimmt. Anschliessend wird aus einer in der Auswerteinheit 22 abgespeicherten Look-Up-Tabelle oder aus Datensätzen den zur ermittelten Farbe zugehörige absolute Dosierwert ausgelesen. Dieser kann mit dem Display 21 dem Benutzer angezeigt werden und/oder direkt mittels der Kommunikationseinheit 23 an einen externen Empfänger gesendet werden. Wird die Dosierhülse 14 zum Einstellen oder Korrigieren einer Dosis gedreht, ändert sich die Farbmarkierung unter dem Farbsensor 31 und der Farbsensor ermittelt die neue Farbe und die Auswerteinheit 22 gibt den entsprechenden Dosierwert aus. Dadurch ist jederzeit eine absolute Bestimmung der eingestellten Dosis möglich.

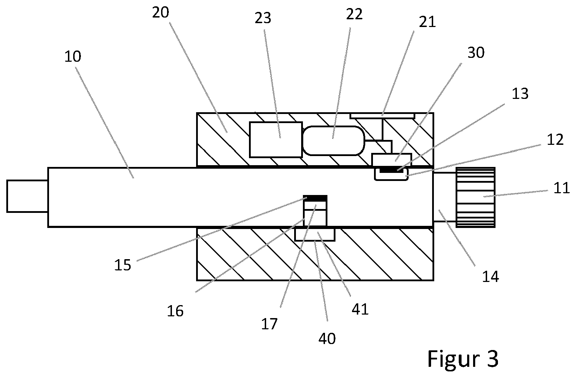

In der Figur 3 ist eine weitere Ausführung der Erfindung ersichtlich. In dieser umfasst das Elektronikmodul 20 zusätzlich zur oben beschriebenen Sensoreinheit 30 zur Erfassung einer Dosis eine zweite Sensoreinheit 40 zur Erfassung einer Absolutposition einer Kolbenstange 17 des Injektionspens 10.

Hierzu weist die Kolbenstange 17 entlang ihrer axialen Länge eine Markierung 15 in der Form von unterschiedliche Farben auf, so dass eine bestimmte Stelle auf der Kolbenstange 17 einer bestimmten Farbe zugeordnet ist. Weiter weist in dieser Ausführung eine Antriebshülse (nicht dargestellt) und das Gehäuse des Injektionspens 10 eine Öffnung 16 auf, so dass von aussen ein Teilbereich der Kolbenstange 17 ersichtlich ist.

Die zweite Sensoreinheit 40 ist wie die oben beschriebene erste Sensoreinheit 30 aufgebaut und umfasst einen zweiten RGB-Farbsensor 41, welcher die unterschiedlichen Farben auf der Kolbenstange 17 erkennen kann. Ein Sensorsignal des zweiten Farbsensors 41 wird in der Auswerteinheit 22 ausgewertet. Hierzu wird mittels einer im Elektronikmodul 20 abgespeicherten Datentabelle die erfasste Farbe einer bestimmten Position der Kolbenstange 17 relativ zum Gehäuse des Injektionspens 10 zugeordnet. Basierend auf der bestimmten Absolutposition der Kolbenstange 17 kann die in der Karpule verbleibende Menge des Medikaments berechnet werden. Diese Restmenge wird dem Benutzer über das Display 21 angezeigt oder mittels der Kommunikationseinheit 23 an einen externen Empfänger weitergeleitet.

### BEZUGSZEICHENLISTE

- 10: Injektionspen
- 11: Dosierknopf
- 12: radiale Öffnung
- 13, 13.1-13.4: Markierung
- 14: Dosierhülse
- 15: Markierung
- 16: Öffnung
- 17: Kolbenstange
- 20: Elektronikmodul
- 21: Display
- 22: Auswerteinheit
- 23: Kommunikationseinheit
- 30: Sensoreinheit
- 31: Farbsensor
- 32: Linse als Diffusor
- 33: LED
- 34: Halterung
- 35: Grundplatte
- 36: Linse
- 40: zweite Sensoreinheit
- 41: Farbsensor

## Patentansprüche

1. Ein Elektronikmodul (20) zum lösbaren Verbinden mit einer Injektionsvorrichtung (10) zum Ausschütten einer Dosis, welches in einem verbundenen Zustand Injektionsereignisse der Injektionsvorrichtung (10) erkennen kann, umfassend eine Sensoreinheit (30) zum Erfassen einer an der Injektionsvorrichtung (10) eingestellten Dosis basierend auf einer optischen Markierung (13), **dadurch gekennzeichnet, dass** die Sensoreinheit (30) einen Farbsensor (31) umfasst, welcher dazu ausgebildet ist, mindestens eine erste, eine zweite und eine dritte Farbe der Markierung (13) eindeutig zu erkennen und
wobei die Sensoreinheit (30) dazu ausgebildet ist, mittels vorbekannten Daten der erkannten ersten, zweiten oder dritten Farbe einen bestimmten absoluten Dosierwert zuzuordnen.

2. Elektronikmodul (20) nach Anspruch 1, **dadurch gekennzeichnet, dass** die Sensoreinheit (30) weiter eine Lichtquelle (33) umfasst, welche dazu ausgebildet ist, eine Oberfläche der zu erfassenden Markierung (13) zu beleuchten.

3. Elektronikmodul (20) nach Anspruch 2, **dadurch gekennzeichnet, dass** die Sensoreinheit (30) eine Linse (32) umfasst, um ein von der Lichtquelle (33) ausgesendetes Licht zu verteilen oder zu fokussieren.

4. Elektronikmodul (20) nach Anspruch 3, **gekennzeichnet durch** eine Grundplatte (35), auf der der Farbsensor (31), die Lichtquelle (33) und die Linse (32) angeordnet sind.

5. Elektronikmodul (20) nach einem der Ansprüche 1 bis 4, **dadurch gekennzeichnet, dass** der Farbsensor (31) ein RGB-Sensor ist.

6. Elektronikmodul nach einem der Ansprüche 1 bis 5, **gekennzeichnet durch** eine Kommunikationseinheit (23), welche mit der Sensoreinheit (30) verbunden ist und dazu ausgebildet ist, den erfassten absoluten Wert an einen externen Empfänger zu übermitteln.

7. Ein Injektionssystem umfassend eine Injektionsvorrichtung (10) und ein Elektronikmodul (20) nach einem der Ansprüche 1 bis 6, wobei die Injektionsvorrichtung (10) ein proportional zu einer einstellbaren Dosis bewegliches Element mit einer optischen Markierung (13) umfasst, **dadurch gekennzeichnet, dass** die Markierung (13) für jede einstellbare Dosis eine Farbe umfasst.

8. Injektionssystem nach Anspruch 7, **dadurch gekennzeichnet, dass** jede Farbe der Markierung (13.1-13.4) einen absoluten Dosierwert repräsentiert.

9. Injektionssystem nach Anspruch 7 oder 8, **dadurch gekennzeichnet, dass** jeder Farbe der Markierung ein eindeutiger Code zugeordnet ist, wobei der Farbsensor dazu ausgebildet ist anhand einer Reihenfolge von Codes von erfassten Farben einen durch die Sensoreinheit bestimmten absoluten Wert einer eingestellten Dosis zu verifizieren.

10. Injektionssystem nach einem der Ansprüche 7 bis 9, **dadurch gekennzeichnet, dass** die Markierung (13.1-13.4) auf einem Dosierelement (14), insbesondere einer Dosierhülse, der Injektionsvorrichtung (10) angeordnet ist.

11. Injektionssystem nach Anspruch 10, **dadurch gekennzeichnet, dass** die Injektionsvorrichtung (10) ein Gehäuse mit einer Öffnung (12) umfasst, wobei durch die Öffnung die Markierung (13.1-13.4) von aussen ersichtlich ist und wobei im verbundenen Zustand der Farbsensor (31) des Elektronikmoduls (20) über der Öffnung (12) angeordnet ist.

12. Injektionssystem nach einem der Ansprüche 7 bis 11, **dadurch gekennzeichnet, dass** die Injektionsvorrichtung eine Kolbenstange mit einer Positionsmarkierung mit mindestens drei unterschiedlichen Farben umfasst und wobei das Elektronikmodul zusätzlich eine zweite Sensoreinheit umfasst zum Erfassen einer absoluten Position der Kolbenstange, wobei die zweite Sensoreinheit einen zweiten Farbsensor umfasst, welcher dazu ausgebildet ist, mindestens eine erste, eine zweite und eine dritte Farbe der Positionsmarkierung eindeutig zu erkennen und wobei die zweite Sensoreinheit dazu ausgebildet ist, mittels vorbekannten Positionsdaten und basierend auf der erkannten Farbe eine absolute Position der Kolbenstange zu bestimmen.

## Claims

1. Electronic module (20) for detachably connecting to an injection device (10) for dispensing a dose, which electronic module, in a connected state, can detect injection events of the injection device (10), the module comprising a sensor unit (30) for capturing a dose set on the injection device (10) based on an optical marking (13), **characterized in that** the sensor unit (30) comprises a color sensor (31) which is designed to clearly detect at least a first, a second and a third color of the marking (13) and
the sensor unit (30) being designed to assign a specific absolute dosing value to the detected first, second or third color by means of previously known data.

2. Electronic module (20) according to claim 1, **characterized in that** the sensor unit (30) further comprises a light source (33) which is designed to illuminate a surface of the marking (13) to be captured.

3. Electronic module (20) according to claim 2, **characterized in that** the sensor unit (30) comprises a lens (32) for distributing or focusing light emitted by the light source (33).

4. Electronic module (20) according to claim 3, **characterized by** a base plate (35) on which the color sensor (31), the light source (33) and the lens (32) are arranged.

5. Electronic module (20) according to any of claims 1 to 4,
**characterized in that** the color sensor (31) is an RGB sensor.

6. Electronic module according to any of claims 1 to 5, **characterized by** a communication unit (23) which is connected to the sensor unit (30) and is designed to transmit the captured absolute value to an external receiver.

7. Injection system comprising an injection device (10) and an electronic module (20) according to any of claims 1 to 6, the injection device (10) comprising an element which is movable proportionally to an adjustable dose and has an optical marking (13), **characterized in that** the marking (13) comprises a color for each adjustable dose.

8. Injection system according to claim 7, **characterized in that** each color of the marking (13.1-13.4) represents an absolute dosing value.

9. Injection system according to claim 7 or claim 8, **characterized in that** each color of the marking is assigned a unique code, the color sensor being designed to verify an absolute value of a set dose determined by the sensor unit on the basis of a sequence of codes of captured colors.

10. Injection system according to any of claims 7 to 9, **characterized in that** the marking (13.1-13.4) is arranged on a dosing element (14), in particular a dosing sleeve, of the injection device (10).

11. Injection system according to claim 10, **characterized in that** the injection device (10) comprises a housing which has an opening (12), the marking (13.1-13.4) being visible from the outside through the opening and, in the connected state, the color sensor (31) of the electronic module (20) being arranged above the opening (12).

12. Injection system according to any of claims 7 to 11, **characterized in that** the injection device comprises a piston rod having a position marking with at least three different colors and the electronic module additionally comprising a second sensor unit for capturing an absolute position of the piston rod, the second sensor unit comprising a second color sensor which is designed to clearly detect at least a first, a second and a third color of the position marking and the second sensor unit being designed to determine an absolute position of the piston rod by means of previously known position data and based on the detected color.

## Revendications

1. Module électronique (20) destiné à être relié de manière amovible à un dispositif d'injection (10) pour délivrer une dose, lequel module électronique, dans un état relié, peut reconnaître des événements d'injection du dispositif d'injection (10), comprenant une unité formant capteur (30) permettant de détecter une dose réglée au niveau du dispositif d'injection (10) sur la base d'un marquage (13) optique, **caractérisé en ce que** l'unité formant capteur (30) comprend un capteur de couleur (31) qui est configuré pour reconnaître de manière univoque au moins une première, une deuxième et une troisième couleur du marquage (13) et
dans lequel l'unité formant capteur (30) est configurée pour associer une valeur de dosage absolue déterminée à la première, deuxième ou troisième couleur reconnue au moyen de données connues au préalable.

2. Module électronique (20) selon la revendication 1, **caractérisé en ce que** l'unité formant capteur (30) comprend en outre une source de lumière (33) qui est conçue pour éclairer une surface du marquage (13) à détecter.

3. Module électronique (20) selon la revendication 2, **caractérisé en ce que** l'unité formant capteur (30) comprend une lentille (32) pour répartir ou focaliser une lumière émise par la source de lumière (33).

4. Module électronique (20) selon la revendication 3, **caractérisé par** une plaque de base (35) sur laquelle sont disposés le capteur de couleur (31), la source de lumière (33) et la lentille (32).

5. Module électronique (20) selon l'une des revendications 1 à 4,
**caractérisé en ce que** le capteur de couleur (31) est un capteur RVB.

6. Module électronique selon l'une des revendications 1 à 5, **caractérisé par** une unité de communication (23) qui est connectée à l'unité formant capteur (30) et qui est configurée pour transmettre la valeur absolue détectée à un récepteur externe.

7. Système d'injection comprenant un dispositif d'injection (10) et un module électronique (20) selon l'une des revendications 1 à 6, dans lequel le dispositif d'injection (10) comprend un élément mobile proportionnellement à une dose réglable et comportant un marquage (13) optique, **caractérisé en ce que** le marquage (13) comprend une couleur pour chaque dose réglable.

8. Système d'injection selon la revendication 7, **caractérisé en ce que** chaque couleur du marquage (13.1-13.4) représente une valeur de dosage absolue.

9. Système d'injection selon la revendication 7 ou 8, **caractérisé en ce qu'un** code unique est associé à chaque couleur du marquage, dans lequel le capteur de couleur est configuré pour vérifier, à l'aide d'une séquence de codes de couleurs détectées, une valeur absolue, déterminée par l'unité formant capteur, d'une dose réglée.

10. Système d'injection selon l'une des revendications 7 à 9,
**caractérisé en ce que** le marquage (13.1-13.4) est disposé sur un élément de dosage (14), en particulier une douille de dosage, du dispositif d'injection (10).

11. Système d'injection selon la revendication 10, **caractérisé en ce que** le dispositif d'injection (10) comprend un boîtier comportant une ouverture (12), dans lequel le marquage (13.1-13.4) est visible depuis l'extérieur à travers l'ouverture et dans lequel le capteur de couleur (31) du module électronique (20) est disposé au-dessus de l'ouverture (12) à l'état relié.

12. Système d'injection selon l'une des revendications 7 à 11,
**caractérisé en ce que** le dispositif d'injection comprend une tige de piston comportant un marquage de position comportant au moins trois couleurs différentes et dans lequel le module électronique comprend en outre une seconde formant capteur permettant de détecter une position absolue de la tige de piston, dans lequel la seconde unité formant capteur comprend un second capteur de couleur qui est configuré pour reconnaître de manière univoque au moins une première, une deuxième et une troisième couleur du marquage de position et dans lequel la seconde unité formant capteur est configurée pour déterminer une position absolue de la tige de piston au moyen de données de position connues au préalable et sur la base de la couleur reconnue.
